# EUROPÄISCHE PATENTSCHRIFT

(11) **EP 0 915 072 B1**
(45) Veröffentlichungstag und Bekanntmachung des Hinweises auf die Patenterteilung: **01.10.2003**
(21) Anmeldenummer: 98119484.8
(22) Anmeldetag: 15.10.1998
(51) Int. Cl.: C07C 6/04, C07C 11/06

(54) **Verfahren zur Herstellung von Propen**
Process for preparing propene
Procédé de préparation de propène

(30) Priorität: 17.10.1997 DE 19746040
(43) Veröffentlichungstag der Anmeldung: 12.05.1999
(73) Patentinhaber: BASF AKTIENGESELLSCHAFT, 67056 Ludwigshafen (DE)
(72) Erfinder: Schwab, Peter, Dr., 67098 Bad Dürkheim (DE); Schulz, Michael, Dr., 67067 Ludwigshafen (DE)
(74) Vertreter: Isenbruck, Günter

(56) Entgegenhaltungen:
- EP-A- 0 742 234
- EP-A- 0 832 867
- DE-A- 1 940 433

## Beschreibung

Die Erfindung betrifft ein Verfahren zur Herstellung von Propen durch Metathese von Olefinen.

Die Olefinmetathese (Disproportionierung) beschreibt in ihrer einfachsten Form die reversible, metallkatalysierte Umalkylidenierung von Olefinen durch Bruch und Neuformierung von C=C-Doppelbindungen. Dabei werden beispielsweise Olefine der Formeln R¹-CH=CH-R² und R³-CH=CH-R⁴ reversibel zu Olefmen der Formeln R¹-CH=CH-R³ und R²-CH=CH-R⁴ umgesetzt. Bei der Metathese acyclischer Olefine unterscheidet man zwischen Selbstmetathese, bei der ein Olefin in ein Gemisch zweier Olefine unterschiedlicher molarer Massen übergeht, und Kreuz- oder Co-Metathese, bei der zwei unterschiedliche Olefine reagieren. Ein Beispiel für eine Selbstmetathese ist die Umsetzung von zwei Molekülen Propen zu Ethen und 2-Buten, wie sie beispielsweise nach dem Phillips -Triolefin- Verfahren ausgeführt wird, siehe Hydrocarbon Processing, Band 46, Nr. 11, November 1967, S. 232. Ein Beispiel für eine Kreuz-Metathese ist die Umsetzung von Propen und 1-Buten zu Ethen und 2-Penten. Ist einer der Reaktionspartner Ethen, so spricht man üblicherweise von einer Ethenolyse.

Die Metathesereaktionen erfolgen in Gegenwart von Katalysatoren. Hierzu eignen sich prinzipiell homogene und heterogene Übergangsmetallverbindungen, insbesondere solche der VI. bis VIII. Nebengruppe des Periodensystems der Elemente, wie auch homogene und heterogene Katalysatorsysteme, in denen diese Verbindungen enthalten sind.

Aus DE-A-19 40 433 ist die Metathese von 1-Buten mit 2-Buten zu Propen und 2-Penten bekannt, wobei Re₂O₇/Al₂O₃ als Katalysator verwendet wird. Das gebildete 2-Penten wird mit Natriumhydrid auf Kaliumcarbonat und Ethen zu Heptenen weiter umgesetzt.

Die Metathese von 1-Buten und 2-Buten zu Propen und 2-Penten wird in K.L. Anderson, T.D. Brown, Hydrocarbon Processing, Band 55, Nr.8, August 1976, S.119-122 als Nebenreaktion bei der Synthese von Isoamylen aufgeführt.

Aus EP-A-0 304 515 ist ein Metatheseverfahren zur Umsetzung von 1-Buten mit 2-Buten zu Propen und Pentenen bekannt, das in einer Reaktivdestillationsvorrichtung mit Re₂O₇/Al₂O₃ als Katalysator ausgeführt wird.

Aus US 3,526,676 ist die Metathese von 1-Buten mit 2-Buten zu Propen und Penten bekannt. Sie wird an MoO₃ und CoO auf Al₂O₃ durchgeführt.

Aus US 3,785,957 ist ein Verfahren zur Herstellung von Treibstoff mit hoher Octanzahl bekannt. Dabei wird ein olefinischer Treibstoff mit Ethen disproportioniert, und der Austrag aufgetrennt wird in einen Propenstrom, einen Butenstrom, einen C₅- oder C₅₋₆-Olefinstrom und einen C₆₊- oder C₇₊-Treibstoffstrom. Der C₅- oder C₅₋₆-Olefinstrom wird mit Ethen an einem WO₃/SiO₂-Festbettkatalysator disproportioniert, um Propen und Butene zu erhalten. Das erhaltene Propen wird zu Ethen und Butenen disproportioniert, und die Butene werden mit Isobutan alkyliert.

Aus US 3,767,565 ist ein Verfahren zur Erhöhung der Octanzahl von Treibstoff bekannt, wobei ein C₅-Schnitt eines olefinischen Treibstoffs mit Ethen in Gegenwart eines Katalysators aus WO₃/SiO₂ und MgO zu Ethen, Propen, n-Butenen und Isobutenen umgesetzt wird. Das erhaltene Propen wird disproportioniert, und die erhaltenen n-Butene werden mit Isobutan alkyliert.

Aus EP-A1-0 691 318 ist ein Olefinmetatheseverfahren bekannt, bei dem C₅-Olefine und Ethen in Gegenwart eines Katalysators zu gemischten C₄-Olefinen und Propen umgesetzt werden. So wird 2-Methyl-2-buten mit Ethen zu Isobuten und Propen umgesetzt. Ein Gemisch aus 2-Pentenen und 2-Methyl-2-buten wird zu einem Gemisch aus 1-Buten, Isobuten und Propen umgesetzt.

Ein Verfahren zur Herstellung von Propen in hoher Ausbeute durch Umsetzung von 1-Buten, 2-Buten und Isobuten ist nicht bekannt.

Ein Verfahren zur Herstellung von Propen in hoher Ausbeute ohne Verwendung eines Überschusses an Ethen ist nicht bekannt. In den vorstehenden Verfahren erfolgt die Herstellung von Propen unter Zudosierung mindestens äquimolarer Mengen an Ethen. Zur Erzielung hoher Propenselektivitäten muß Ethen in großer Menge im Kreis gefahren werden. Zudem wird im Feed enthaltenes Isobuten nicht umgesetzt und vermindert die Raum-Zeit-Ausbeute.

Eine Aufgabe der Erfindung ist die Bereitstellung eines Verfahrens zur Herstellung von Propen in hoher Ausbeute aus 1-Buten, 2-Buten und Isobuten enthaltenden Gemischen. Dabei soll nicht mit einem Ethen-Überschuß gearbeitet werden müssen. Die Gewinnung von Propen aus 1-Buten-armen C₄-Strömen soll mit möglichst geringem Ethenbedarf möglich sein, wobei auch Isobuten zu Wertprodukt umgesetzt werden soll.

Die Aufgabe wird erfindungsgemäß gelöst durch ein Verfahren zur Herstellung von Propen durch
a) Umsetzung von 1-Buten, 2-Buten und Isobuten, wobei das Verhältnis von Molanteil 1-Buten und 2-Buten zu Isobuten 10:1 bis 1:5 beträgt, zu Propen, 2-Penten und 2-Methyl-2-buten in Gegenwart eines Metathesekatalysators, der mindestens eine Verbindung eines Metalls VIb, VIIb oder VIII Nebengruppe des Periodensystems der Elemente enthält,
b) anschließendes Trennen des gebildeten Propens und 2-Pentens / 2-Methyl-2-butens,
c) anschließende Umsetzung des 2-Pentens und 2-Methyl-2-butens mit Ethen zu Propen, 1-Buten und Isobuten in Gegenwart eines Metathesekatalysators, der mindestens eine Verbindung eines Metalls der VIb, VIIb oder VIII Nebengruppe des Periodensystems der Elemente enthält,
d) anschließendes Trennen des gebildeten Propens und 1-Butens/Isobutens,
e) anschließendes Rückführen des gebildeten 1-Butens und Isobutens in Schritt a).

Das erfindungsgemäße Verfahren beinhaltet 2 Metatheseschritte. Im ersten Schritt werden 1-Buten, 2-Buten und Isobuten zu Propen, 2-Penten und 2-Methyl-2-buten umgesetzt. In einem zweiten Schritt werden dann 2-Penten und 2-Methyl-2-buten mit Ethen zu 1-Buten, Isobuten und Propen umgesetzt. 1-Buten und Isobuten werden gemäß einer Ausführungsform der Erfindung dabei in den ersten Reaktionsschritt zurückgeführt.

Als Nettoreaktion ergibt sich somit die Umsetzung von 2-Buten mit Ethen zu 2 Molekülen Propen. Bei der Umsetzung von 2-Penten und 2-Methyl-2-buten mit Ethen werden gemäß einer Ausführungsform der Erfindung formal nur equimolare Mengen an Einsatzstoffen benötigt, um mit hoher Ausbeute zu den Produkten zu gelangen. Somit kann im Gegensatz zum umgekehrten Triolefin-Verfahren die eingesetzte Ethenmenge geringgehalten werden.

Beide Metatheseschritte können als Reaktivdestillation ausgeführt werden, wie sie nachstehend beschrieben ist.

1-Buten, 2-Buten und Isobuten können in der Umsetzung als reine Stoffe eingesetzt werden gemäß einer Ausführungsform der Erfindung. Gemäß einer anderen Ausführungsform der Erfindung werden die Butene in Form eines C₄-Stroms eingesetzt, der beispielsweise aus einem Cracker, insbesondere Steamcracker, oder einer Raffination stammt. Der C₄-Strom kann dabei neben den Butenen C₄-Alkane enthalten. Gemäß einer Ausführungsform der Erfindung wird ein C₄-Strom verwendet, der aus Raffinat I besteht. Raffinat I ist dabei eine Fraktion aus 1-Buten, cis/trans-2-Buten, Isobuten sowie n-Butan und Isobutan. Beispielsweise kann Raffinat I 60 - 90 Gew.-% Olefine und 10 - 40 Gew.-% Butane enthalten, mit beispielsweise 10 - 40 Gew.-% 1-Buten, 10 - 40 Gew.-% 2-Buten, 10 - 50 Gew.-% Isobuten. Gemäß einer Ausführungsform der Erfindung besitzt der eingesetzte C₄-Strom einen Anteil an Butenen von 20 bis 100, vorzugsweise 50 bis 90, insbesondere 70 bis 90 Gew.-%. Das Verhältnis von 1-Buten zu 2-Buten beträgt dabei 10:1 bis 1:10, vorzugsweise 3:1 bis 1:3, insbesondere 2:1 bis 1:2. Das Verhältnis von Molanteil 1-Buten + 2-Buten zu Isobuten beträgt vorzugsweise 10:1 bis 1:5, insbesondere 3:1 bis 1:2. Gemäß einer Ausführungsform der Erfindung kann der C₄-Strom geringe Mengen anderer Kohlenwasserstoffe enthalten.

Vorzugsweise wird Raffinat I aus dem beim Steamcracken erhaltenen Roh-C₄-Schnitt nach Abtrennung von Butadien erhalten. Dies geschieht entweder durch Butadien-Extraktion mit polar-aprotischen Solventien (z.B. N-Methylpyrrolidon) oder durch Selektivhydrierung. Je nach Aufarbeitung werden unterschiedliche C₄-Komponentenverteilungen erhalten, beispielsweise (Angaben in Gewichts-%):

| | nach C₄H₆-Extraktion | nach C₃H₄-Selektivhydrierung |
|---|---|---|
| 1-Buten | 26% | 33% |
| cis/trans-2-Buten | 15% | 30% |
| i-Buten | 46% | 24% |
| Butadien | 100 ppm | 2000 ppm |
| Butane | 13% | 13% |

Gemäß einer Ausführungsform der Erfindung kann als Ausgangsstoff jeder Strom verwendet werden, der 1-Buten, 2-Buten und Isobuten enthält, wobei das Verhältnis von Molanteil 1-Buten und 2-Buten zu Isobuten 10:1 bis 1:5 beträgt. Gemäß einer Ausführungsform der Erfindung können dabei das 1-Buten und Isobuten aus der erfindungsgemäßen Synthese selbst stammen und mit zugeführtem 2-Buten gemischt werden.

Der eingesetzte C₄-Feedstrom wird vorzugsweise vor dem Einsatz im erfindungsgemäßen Verfahren vorgereinigt, um gegebenenfalls vorliegende Spuren an Wasser, Sauerstoff bzw. Oxygenates, Schwefel oder Schwefel enthaltenden Verbindungen, Stickstoff, Phosphor oder Halogen speziell Chloriden zu entfernen. Vorzugsweise erfolgt die Entfernung durch Leiten des C₄-Feedstroms über Adsorbermaterialien, wie Zeolithe und zeolithanaloge Phosphate, hochoberflächige Oxide des Siliziums, Aluminiums, Titans, Zirkons, Bleicherden, Tone, Hydrotalcite, hochoberflächige Phosphate, Aktivkohlen und Kohlenstoffmolekularsiebe, sowie organische Polymere und Ionenaustauscherharze, bevorzugt NaX-Molsieb. Die Adsorbermaterialien liegen vorzugsweise als Schutzbett (guard bed) vor.

Einsetzbare Verfahren zur Adsorption und adsorptiven Reinigung sind beispielsweise beschrieben in W. Kast, Adsorption aus der Gasphase, VCH, Weinheim (1988). Der Einsatz von zeolithischen Adsorbentien wird erläutert bei D.W. Breck, Zeolite Molecular Sieves, Wiley, New York (1974). Die Entfernung von speziell Acetaldehyd aus C₃ bis C₁₅-Kohlenwasserstoffen in flüssiger Phase ist im EP-A-0 582 901 beschrieben. Die in der vorstehenden Literatur angeführten Verfahren können vorliegend eingesetzt werden. So bringt man vorzugsweise den Eduktstrom in gasförmiger, flüssiger oder überkritischer Phase mit den Adsorbentien in Kontakt.

Neben der Umsetzung von 1-Buten, 2-Buten und Isobuten zu Propen und 2-Penten sowie 2-Methyl-2-buten kann ein geringer Anteil an 3-Hexen, Ethen, 2,3-Dimethyl-2-buten und 2-Methyl-2-penten als Nebenprodukt erhalten werden. Zudem können auch geringe 2-Methyl-2-penten als Nebenprodukt erhalten werden. Zudem können auch geringe Mengen höhersiedender Verbindungen vorliegen:

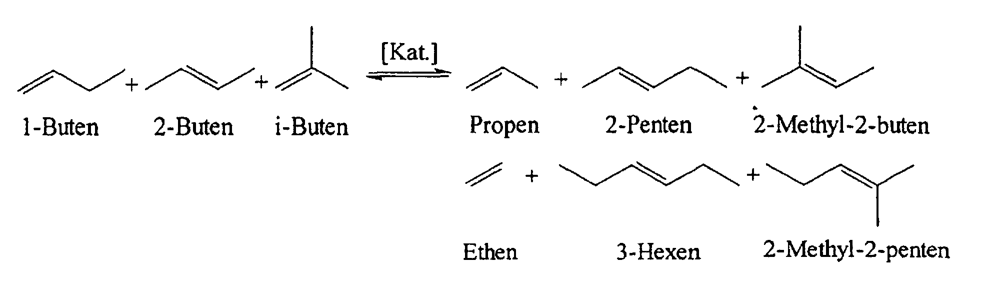

Das Zielmolekül Propen entsteht in obiger Reaktion durch Kreuzmetathesereaktion der á-Olefine 1-Buten und i-Buten mit 2-Buten. Als Coprodukt fallen in annähernd äquimolaren Mengen die C5-Olefine 2-Penten und 2-Methyl-2-buten an. Deutlich benachteiligt sind demgegenüber die jeweiligen Selbstmetathesereaktionen von 1-Buten und Isobuten (zu Ethen und 3-Hexen bzw. 2,3-Dimethyl-2-buten) sowie die Kreuzmetathese der beiden á-Olefine (zu 2-Methyl-2-penten). Die Bildung des Isobuten-Selbstmetatheseprodukts 2,3-Dimethyl-2-buten wurde nicht beobachtet.

Die in diesem ersten Schritt erhaltenen C4⁺-Olefine können nach destillativer Abtrennung von Ethen und Propen als Cocrack-Feed mit einem verglichen mit dem Ausgangsmaterial deutlich höheren Crackwert fungieren. Alternativ ist nach Trennung von C4 und C5⁺ eine Erhöhung der Propenausbeute durch die beschriebene nachfolgende Ethenolyse von C5⁺ möglich. Als Nebenkomponenten fallen bei dieser Umsetzung vorzugsweise ausschließlich 1-Buten und i-Buten an, welche in den ersten Metatheseschritt zurückgeführt oder alternativ nach bekannter Technologie als Reinstoffe isoliert werden können.

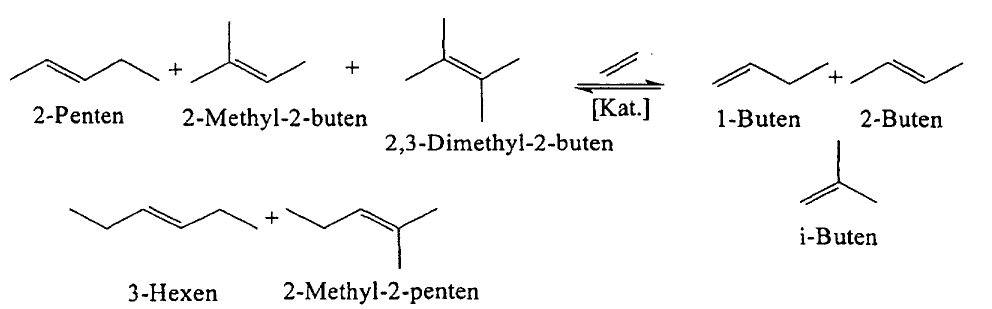

Die geringen Mengen an Nebenprodukten im ersten Schritt, die gemäß einer Ausführungsform der Erfindung 1 bis 20 Gew.-%, vorzugsweise 1 bis 5 Gew.-%, bezogen auf die Mengen an gebildetem 2-Penten und 2-Methyl-2-buten ausmachen, stören bei der nachfolgenden Umsetzung (Ethenolyse) nicht, so daß gemäß einer Ausführungsform der Erfindung keine Reinigung des 2-Pentens und 2-Methyl-2-butens von diesen Nebenprodukten vor der weiteren Umsetzung erforderlich ist. Gemäß einer Ausführungsform der Erfindung werden das 2-Penten und 2-Methyl-2-buten in reiner Form in der zweiten Umsetzung eingesetzt.

Unter dem Ausdruck "2-Penten und 2-Methyl-2-buten" werden auch solche Gemische verstanden, die neben 2-Penten und 2-Methyl-2-buten geringe Mengen an Hexenen, insbesondere 3-Hexen, und anderen höhersiedenden Verbindungen enthalten.

Entsprechend wird unter dem Ausdruck "Butene", wie "1-Buten", "2-Buten" und "Isobuten" auch ein Gemisch verstanden, das neben dem Buten oder den Butenen C₄-Alkane, insbesondere Butane enthält.

Mehrere Ausführungsformen der Erfindung werden nachstehend anhand der Zeichnung erläutert, in der
- Figur 1: eine schematische Darstellung einer Ausführungsform des erfindungsgemäßen Verfahrens zeigt,
- Figur 2: eine weitere Ausführungsform des erfindungsgemäßen Verfahrens zeigt,
- Figur 3: eine weitere Ausführungsform des erfindungsgemäßen Verfahrens zeigt.

Dabei bedeuten die in den Figuren verwendeten Abkürzungen folgendes:
- Bu:: Butane
- Et:: Ethen
- Pr:: Propen
- H:: Hochsieder
- I:: Raffinat I
- C4:: C4-Olefine
- C5⁺:: Olefine mit 5 oder mehr Kohlenstoffatomen
- R1:: Reaktor
- R2:: Reaktor
- D1:: Destillationskolonne (wenn unter D1 ein senkrechter Strich vorgesehen ist, handelt es sich um eine Trennwandkolonne)
- D2:: Kolonne (wenn unter D2 ein senkrechter Strich vorgesehen ist, handelt es sich um eine Trennwandkolonne)
- D3:: Destillationskolonne

Nachstehend wird eine Ausführungsform des erfindungsgemäßen Verfahrens beschrieben, umfassend
a) Umsetzung von 1-Buten, 2-Buten und Isobuten, wobei das Verhältnis von Molanteil 1-Buten und 2-Buten zu Isobuten 10:1 bis 1:5 beträgt zu Propen, 2-Penten und 2-Methyl-2-buten in Gegenwart eines Metathesekatalysators, der mindestens eine Verbindung eines Metalls VIb, VIIb oder VIII Nebengruppe des Periodensystems der Elemente enthält,
b) anschließendes Trennen des gebildeten Propens und 2-Pentens/2-Methyl-2-butens,
c) anschließende Umsetzung des 2-Pentens und 2-Methyl-2-butens mit Ethen zu Propen, 1-Buten und Isobuten in Gegenwart eines Metathesekatalysators, der mindestens eine Verbindung eines Metalls der VIb, VIIb oder VIII Nebengruppe des Periodensystems der Elemente enthält,
d) anschließendes Trennen des gebildeten Propens und 1-Butens/Isobutens,
e) anschließendes Rückführen des gebildeten 1-Butens und Isobutens in Schritt a).

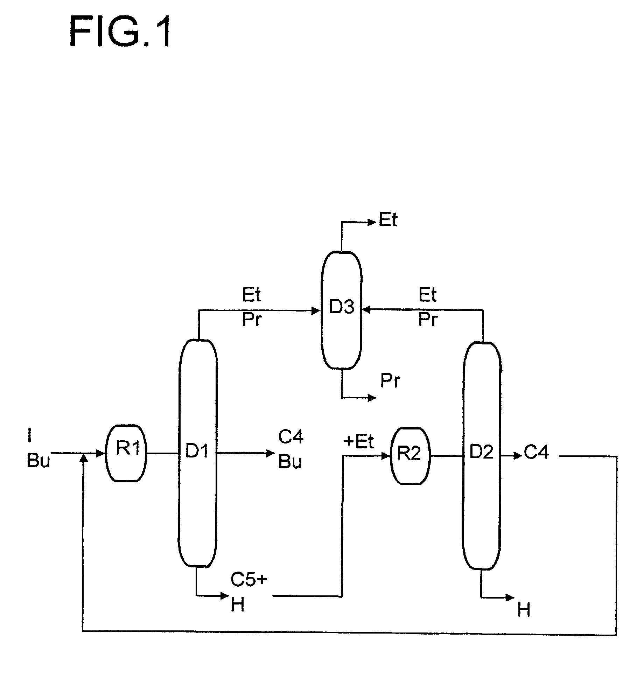

Diese Ausführungsform ist in Fig. 1 dargestellt.

In einem ersten Reaktor R1 werden in Gegenwart des erfindungsgemäßen Metathesekatalysators 1-Buten, 2-Buten und Isobuten zu Propen, 2-Penten und 2-Methyl-2-buten umgesetzt. Dazu wird ein Raffinat I-Strom in den Reaktor geführt. An den Reaktor schließt sich eine Destillationskolonne D1 an, an deren Kopf Propen und als Nebenprodukt gebildetes Ethen entfernt werden. Nicht umgesetztes Raffinat I wird im Mittelabzug entfernt. Es kann auch teilweise in den Reaktor R1 zurückgeführt werden (in Fig. 1 nicht dargestellt). Aus dem Sumpf von D1 werden 2-Penten, 2-Methyl-2-buten und als Nebenprodukt gebildetes 3-Hexen sowie Hochsieder entnommen. Der Sumpf wird sodann zusammen mit eingespeistem Ethen einem Reaktor R2 zugeführt, der wiederum einen erfindungsgemäßen Metathesekatalysator enthält. In diesem Reaktor R2 findet die Umsetzung von 2-Penten und 2-Methyl-2-buten mit Ethen zu 1-Buten, Isobuten und Propen statt. Der Austrag aus Reaktor R2 wird einer Destillationskolonne D2 zugeführt, an deren Kopf Propen und nicht umgesetztes Ethen abgeführt werden. Im Mittelabzug werden gebildetes 1-Buten und Isobuten ausgetragen, und vorzugsweise zumindest teilweise zum Reaktor R1 zurückgeführt. Im Sumpf von D2 fallen nicht umgesetztes 2-Penten, 2-Methyl-2-buten sowie als Nebenprodukte 3-Hexen und Hochsieder an. Diese werden vorzugsweise in den Reaktor R2 zurückgeführt. Die aus D1 und D2 am Kopf abgeführten Gemische aus Propen und Nebenprodukt Ethen werden in einer weiteren Destillationskolonne D3 aufgetrennt. Am Kopf von D3 wird Ethen gewonnen, das vorzugsweise in den Reaktor R2 (zurück)geführt wird (nicht in Fig. 1 dargestellt), oder als Co-Crackfeed ausgeschleust wird. Das im Sumpf von D3 erhaltene Propen ist das gewünschte Umsetzungsprodukt des erfindungsgemäßen Verfahrens. D1 und D2 sind so ausgelegt, daß am Kopf der Kolonne eine leichtsiedende Phase, insbesondere eine C_{2/3}-Phase, die Ethen und Propen enthält, abgeführt wird. Als Mittelsiederphase werden C₄-Ströme abgeführt, insbesondere Butene und Butane. In der Sumpfphase werden C_{≥5}-Kohlenwasserstoffe ausgetragen.

Die Reaktoren R1 und R2 können beliebige geeignete Reaktoren sein. Sie können zur kontinuierlichen oder diskontinuierlichen Reaktionsführung dienen. Somit können Sie gemäß einer Ausführungsform Druckgefäße, wie Druckglasgefäße, gemäß einer weiteren Ausführungsform Rohrreaktoren sein.

Gemäß einer Ausführungsform der Erfindung beträgt der Gesamtumsatz in R1 20 bis 90, vorzugsweise 50 bis 80%.

Gemäß einer Ausführungsform der Erfindung beträgt der Gesamtumsatz in R2 20 bis 100, vorzugsweise 60 bis 90%.

Vorzugsweise findet die Umsetzung in R1 in flüssiger Phase statt. Dabei werden Druck und Temperatur so gewählt, daß die Reaktionspartner in der flüssigen Phase verbleiben.

Gemäß einer Ausführungsform der Erfindung beträgt die Temperatur in R1 0 bis 150°C, vorzugsweise 20 bis 80°C. Der Druck beträgt gemäß einer Ausführungsform der Erfindung 2 bis 200 bar, vorzugsweise 5 bis 20 bar. Die Umsetzung in R2 (Ethenolyse) wird gemäß einer Ausführungsform der Erfindung bei Temperaturen von 20 bis 150°C, vorzugsweise 20 bis 80°C unter einem Ethendruck von 5 bis 200 bar, vorzugsweise 30 bis 80 bar durchgeführt. Ethen kann dabei kontinuierlich nachgepreßt werden, so daß ein konstanter Druck eingehalten wird.

Die Umsetzungen in R1 und R2 können für eine Zeit von einer Sekunde bis 10 Stunden, vorzugsweise 1 bis 60 Minuten kontinuierlich oder diskontinuierlich in Reaktoren wie Druck(glas)gefäßen, Rohrreaktoren oder Reaktionskolonnen erfolgen. Bevorzugt werden Rohrreaktoren eingesetzt.

Bei den Destillationskolonnen D1 und D2 handelt es sich gemäß einer Ausführungsform der Erfindung um solche Kolonnen, die eine Auftrennung eines Kohlenwasserstoffstroms in C_{2/3}-Ströme, C₄-Ströme und C_{≥5}-Ströme erlauben. Die Kolonnen können als Trennwandkolonnen ausgeführt sein. D3 ist gemäß einer Ausführungsform der Erfindung eine Kolonne, die die Trennung von Ethen und Propen erlaubt. Gemäß einer Ausführungsform der Erfindung ist der Reaktor R1 mit der Destillationskolonne D1 kombiniert zu einer Reaktivdestillationseinrichtung. Dabei erfolgt die Umsetzung direkt in der Destillationskolonne. Der Katalysator liegt in der Reaktionskolonne vor, so daß gleichzeitig mit der Umsetzung oder unmittelbar darauffolgend die Destillation ausgeführt wird. Ein entsprechendes Verfahren ist unter der Bezeichnung "Reaktivdestillation" bekannt.

Gemäß einer Ausführungsform sind Reaktor R2 und Destillationskolonne D2 zusammengefaßt zu einer Reaktivdestillationsvorrichtung, bei der Umsetzung und Destillation kombiniert sind entsprechend der vorstehend beschriebenen Reaktivdestillation.

Gemäß einer Ausführungsform der Erfindung finden beide Umsetzungsreaktionen in Reaktivdestillationsvorrichtungen statt. Bei beiden Umsetzungen handelt es sich um Gleichgewichtsreaktionen, so daß gemäß einer Ausführungsform der Erfindung die Verfahrensprodukte zur Erzielung eines möglichst hohen Umsatzes möglichst schnell aus dem Gleichgewicht entfernt werden. Dies ist insbesondere bei Verwendung von Reaktivdestillationsvorrichtungen möglich.

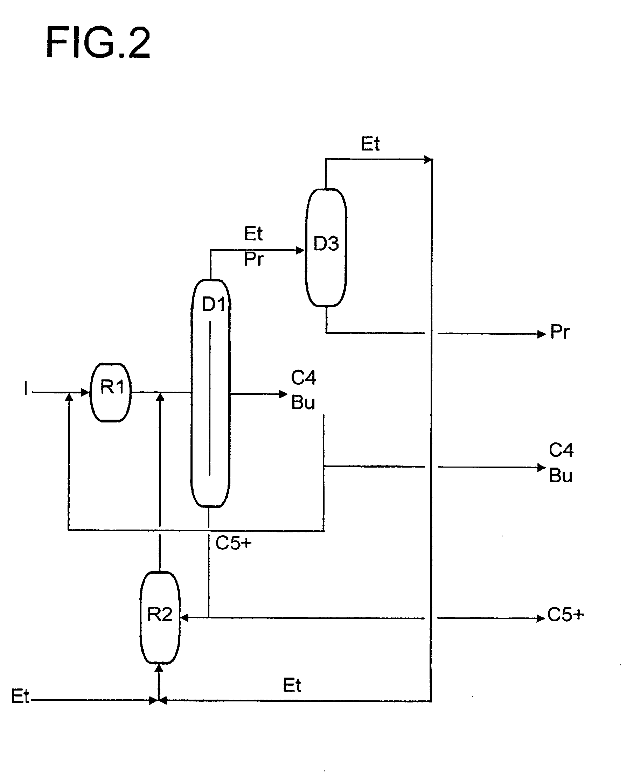

Anstelle einer normalen Destillationskolonne D1 kann eine Trennwandkolonne vorgesehen werden. Ein derartiges Verfahren ist in Figur 2 dargestellt. Das dargestellte Verfahren ist zudem gegenüber dem in Figur 1 gezeigten Verfahren modifiziert.

Wie in der vorstehend beschriebenen Ausführungsform wird im Reaktor R1 eine Metathese an einem heterogenen Metathesekatalysator durchgeführt, wobei Raffinat I eingesetzt wird. Die Destillationskolonne D1 dient der Auftrennung der bei der Metathese entstandenen Reaktionsprodukte. Die Destillationskolonne D3 dient der Trennung von Ethen und Propen. Der Reaktor R2 dient der Umsetzung von C₅⁺-Hochsiedern mit Ethen.

Im Unterschied zur vorstehenden Ausführungsform ist die Destillationskolonne D1 als Trennwandkolonne ausgeführt. Zudem wird der Mittelsiederaustrag aus D1, der C₄-Olefine und Butane enthält, teilweise in den Feedstrom von Raffinat I zurückgeführt. Da die Destillationskolonnen D1 und D2 die gleiche Trennaufgabe bewältigen müssen, wurde in dieser Ausführungsform nur eine derartige Destillationskolonne D1 vorgesehen. Damit läßt sich der apparative Aufwand verringern. Entsprechend wurde das Reaktionsschema angepaßt: Der Hochsiederaustrag aus D1 wird in den Reaktor R2 geführt oder teilweise ausgeschleust. Der Austrag aus dem Reaktor R2 wird in die Destillationskolonne D1 geführt. Die Beschickung des Reaktors R2 mit Ethen erfolgt zum einen aus dem Leichtsiederaustrag der Destillationskolonne D3, zum anderen durch zusätzlich eingeführtes Ethen. Aus dem Verfahren werden Propen als Hauptprodukt und zudem C₄-Olefine und C₅⁺-Olefine ausgeschleust.

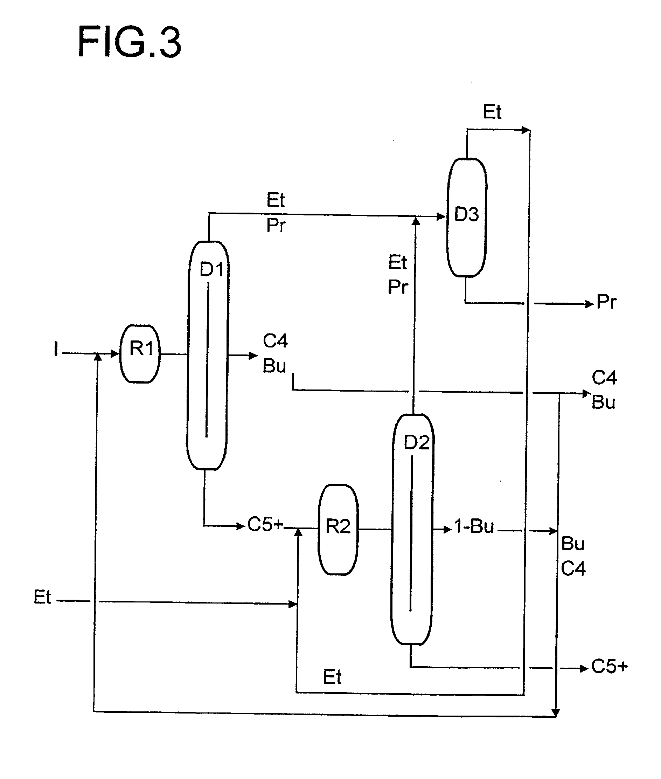

In Figur 3 ist eine Ausführungsform des erfmdungsgemäßen Verfahrens dargestellt, die der in Figur 1 dargestellten Ausführungsform weitgehend entspricht. Die Kolonne D2 ist ebenso wie die Kolonne D1 als Trennwandkolonne ausgelegt.

Im Unterschied zu dem Verfahren gemäß Figur 1 wird der Mittelsiederaustrag aus D1, der C₄-Olefine und Butane enthält, teilweise ausgeschleust oder mit dem Mittelsiederaustrag aus der Kolonne D2 vereint und in den Reaktor R1 zurückgeführt. Das aus der Destillationskolonne D3 gewonnene Ethen wird in den Reaktor R2 geführt, indem die Umsetzung mit der C₅⁺-Fraktion erfolgt. Es werden wiederum Propen als Hauptprodukt und Teile der C₄-Olefinfraktion und Butane sowie die C₅⁺-Fraktion aus der Destillationskolonne D2 (auch als Co-Crackfeed) ausgeschleust.

### KATALYSATOR

In den erfindungsgemäßen Verfahren können alle geeigneten Metathesekatalysatoren eingesetzt werden.

Gemäß einer Ausführungsform der Erfindung ist der Katalysator ein heterogener Katalysator, insbesondere ein Trägerkatalysator. Gemäß einer Ausführungsform der Erfindung enthält der Katalysator mindestens eine Verbindung eines Metalls der VIb., VIIb. oder VIII. Nebengruppe des Periodensystems der Elemente. Vorzugsweise enthält der Katalysator eine Rutheniumverbindung und/oder Rheniumverbindung. Derartige Katalysatoren sind beispielsweise beschrieben in K.J. Ivin, I.C. Mol, Olefin Metathesis and Metathesis Polymerization, 2^{nd} edition, Academic Press, New York, 1996; G.W. Parshall, S.D. Ittel, Homogeneous Catalysis, 2. Aufl., 1992, John Wiley & Sons, New York, Chichester, Brisbane, Toronto, Singapur, S. 217ff; R.H. Grubbs in Prog. Inorg. Chem., S. Lippard (Hrsg.), John Wiley & Sons, New York, 1978, Vol.24, 1-50; R.H. Grubbs in Comprehensive Organomet. Chemie., G. Wilkinson (Hrsg.), Pergamon Press, Ltd., New York, 1982, Vol. 8, 499-551; D.S. Breslow, Prog. Polym. Sci. 1993, Vol. 18, 1141-1195.

Gemäß einer Ausführungsform der Erfindung handelt es sich bei der Metallverbindung um ein Metalloxid, Teiloxid mit zusätzlich vorliegenden organischen Resten oder um eine Carbonylverbindung.

Gemäß einer Ausführungsform der Erfindung wird ein homogener Katalysator eingesetzt. Der Katalysator ist dabei mindestens eine Verbindung eines Metalls der VIb., VIIb. oder VIII. Nebengruppe des Periodensystems der Elemente. Vorzugsweise wird Rhenium oder Ruthenium in den Metallverbindungen eingesetzt.

Gemäß einer Ausführungsform der Erfindung werden Rutheniumverbindungen eingesetzt, wie sie in WO 93/20111 und WO 96/04289 sowie J.Chem. Soc., Chem. Commun. 1995, 1127 bis 1128 beschrieben sind.

Gemäß einer bevorzugten Ausführungsform der Erfindung wird RuX₂(CHR)(PR'₃)₂ eingesetzt, wobei die Reste R und R' C₁₋₁₂-Alkylreste, vorzugsweise C₁₋₆-Alkylreste oder C₆₋₁₂-Arylreste sind, R' besonders bevorzugt ein C₃₋₈-Cycloalkylrest, insbesondere C₅- oder C₆-Cycloalkylrest ist und X ein Halogenid ist, wie Chlorid, Bromid oder Iodid.

Insbesondere wird erfindungsgemäß RuCl₂(=CHPh)(PCy₃)₂ eingesetzt, gemäß einer Ausführungsform der Erfindung als Lösung, beispielsweise in Methylenchlorid.

Vorzugsweise ist die Metallverbindung ein Oxid von Molybdän, Wolfram oder vorzugsweise Rhenium, insbesondere Re₂O₇.

### TRÄGER

Die erfindungsgemäßen Katalysatoren enthalten gemäß einer Ausführungsform der Erfindung einen Träger. Als Träger kommen dabei insbesondere anorganische Träger zur Anwendung, wie Al₂O₃, insbesondere ã-Al₂O₃, SiO₂, Fe₂O₃, oder deren Gemische, wie SiO₂/Al₂O₃, B₂O₃/SiO₂/Al₂O₃ oder Fe₂O₃/Al₂O₃.

Der Metalloxidgehalt auf dem Träger beträgt dabei gemäß einer Ausführungsform der Erfindung 1 bis 20 Gew.-%, vorzugsweise 3 bis 15 Gew.-%, insbesondere 8 bis 12 Gew.-%, bezogen auf das Gesamtgewicht des Trägerkatalysators.

Beispielsweise können Re₂O₇ auf Al₂O₃, ein bereits bei sehr milden Reaktionsbedingungen von 20 bis 80°C aktiver Katalysator, oder MO₃/SiO₂ mit M = Mo, W bei höherer Reaktionstemperatur eingesetzt werden.

Bevorzugt wird als Katalysator Re₂O₇ auf Al₂O₃, SiO₂/Al₂O₃, SiO₂/Al₂O₃/Fe₂O₃ oder B₂O₃/Al₂O₃ verwendet. Dabei beträgt der Anteil an Metalloxid vorzugsweise 1 bis 20 Gew.-%, besonders bevorzugt 3 bis 10 Gew.-%. Gemäß einer Ausführungsform der Erfindung wird MeReO₃ anstelle von Re₂O₇ oder im Gemisch damit verwendet.

Besonders bevorzugt wird erfindungsgemäß Re₂O₇ auf Al₂O₃ verwendet.

Die Katalysatoren werden gemäß einer Ausführungsform der Erfindung frisch calciniert eingesetzt, wobei sie keiner weiteren Aktivierung, beispielsweise durch Alkylierungsmittel bedürfen. Desaktivierte Katalysatoren können erfindungsgemäß durch Abbrennen von Coke-Rückständen beispielsweise bei 550°C im Luftstrom und Abkühlung unter Argon regeneriert werden.

Die erfindungsgemäßen Umsetzungen können dabei in Gegenwart eines Lösungsmittels, beispielsweise eines Kohlenwasserstofflösungsmittels durchgeführt werden. Gemäß einer bevorzugten Ausführungsform der Erfindung werden die Umsetzungen ohne weiteres zugesetztes Lösungsmittel durchgeführt.

Nachstehend wird die Erfindung anhand von Beispielen näher erläutert.

### BEISPIELE

Kontinuierliche Versuche zur Synthese von Propen aus Raffinat I.

### Beispiele 1 bis 3

Kontinuierliche Versuche zur Metathese von i-Buten enthaltenden C4-Fraktionen Raffinat I wurde bei 60°C und 10 bar kontinuierlich bei unterschiedlichen Verweilzeiten über ein mit einem Re₂O₇/Al₂O₃-Heterogenkontakt bestücktes Strömungsrohr geleitet. Der Reaktionsaustrag wurde nach Entspannung gaschromatographisch untersucht. Die Ergebnisse zeigt Tabelle 1.

**Tabelle 1**

| Beispiel | VWZ (min) | Umsatz 1-Buten | Umsatz i-Buten | Umsatz 2-Butene | Umsatz Gesamt-Butene | RZA Propen |
|---|---|---|---|---|---|---|
| 1 | 10 min | 65 % | 39 % | 42 % | 49 % | 550 g/l-h |
| 2 | 5 min | 65% | 39% | 46% | 50% | 970 g/l-h |
| 3 | 3 min | 58 % | 30 % | 40% | 43 % | 1200 g/l-h |
| RZA: Raum-Zeit-Ausbeute | | | | | | |
| VWZ: Verweilzeit | | | | | | |

### Beispiele 4 bis 5

Kontinuierliche Versuche zur Ethenolyse des C5/C6-Hochsiederaustrages Der in den Beispielen 1 bis 3 erhaltene Hochsiederaustrag, bestehend aus 2-Penten, 2-Methyl-2-buten, 3-Hexen und 2-Methyl-2-penten, wurde bei 60°C und 50 bzw. 70 bar Ethen (Eduktverhältnis C2 : C5, C6 = 1 : 1) kontinuierlich über ein mit Re₂O₇/Al₂O₃-Heterogenkontakt bestücktes Strömungsrohr geleitet. Der Reaktionsaustrag wurde nach Entspannung gaschromatographisch untersucht. Die Ergebnisse zeigt Tabelle 2.

**Tabelle 2**

| Beispiel | Umsatz 2-Penten | Umsatz 2-Me-2-buten | Umsatz 3-Hexen | Umsatz 2-Me-2-Penten | Selektivität Propen |
|---|---|---|---|---|---|
| 4 | 89% | 83% | 87% | 85% | 91% |
| 5 | 92% | 85% | 90% | 88% | 93% |

## Patentansprüche

1. Verfahren zur Herstellung von Propen durch
a) Umsetzung von 1-buten, 2-Buten und Isobuten, wobei das Verhältnis von Molanteil 1-Buten + 2-Buten zu Isobuten 10:1 bis 1:5 beträgt, zu Propen, 2-Penten und 2-Methyl-2-buten in Gegenwart eines Metathesekatalysators, der mindestens eine Verbindung eines Metalls der VIb., VIIb. oder VIII. Nebengruppe des Periodensystems der Elemente enthält;
b) anschließendes Trennen des gebildeten Propens und 2-Pentens/2-Methyl-2-butens,
c) anschließende Umsetzung des 2-Pentens und 2-Methyl-2-butens mit Ethen zu Propen, 1-Buten und Isobuten in Gegenwart eines Metathesekatalysators, der mindestens eine Verbindung eines Metalls der VIb., VIIb. oder VIII. Nebengruppe des Periodensystems der Elemente enthält,
d) anschließendes Trennen des gebildeten Propens und 1-Butens/Isobutens,
e) anschließendes Rückführen des gebildeten 1-Butens und Isobutens in Schritt a).

2. Verfahren nach Anspruch 1, wobei Schritt b) eine Destillation ist, die in einer Trennwandkolonne durchgeführt werden kann, in der eine Propen enthaltendes Leichtsiederphase, gegebenenfalls eine Butene enthaltende Mittelsiederphase und eine 2-Penten und 2-Methyl-2-buten enthaltende Sumpfphase erhalten werden,
und/oder
wobei Schritt d) eine Destillation ist, die in einer Trennwandkolonne durchgeführt werden kann, in der eine Propen enthaltende Leichtsiederphase, eine 1-Buten und Isobuten enthaltende Mittelsiederphase und gegebenenfalls eine 2-Penten und 2-Methyl-2-buten enthaltende Sumpfphase erhalten werden, wobei Schritte b) und d) in einer Destillationskolonne durchgeführt werden können.

3. Verfahren nach Anspruch 1 oder 2, wobei die Umsetzung in Schritten a) und/oder c) nicht vollständig erfolgt und in Schritt b) und/oder d) eine C_{2/3} enthaltende Leichtsiederphase, eine C₄-Mittelsiederphase und eine C_{≥5} enthaltende Sumpfphase erhalten werden,
wobei die gegebenenfalls zusammengefaßten Leichtsiederphasen durch Destillation in C₂- und C₃-Phasen getrennt werden und die C₂-Phase in Schritt c) zurückgeführt wird,
die gegebenenfalls zusammengefaßten Mittelsiederphasen zumindest teilweise in Schritt a) zurückgeführt werden und die gegebenenfalls zusammengefaßten Sumpfphasen zumindest teilweise in Schritt c) zurückgeführt werden.

4. Verfahren nach einem der Ansprüche 1 bis 3, wobei 1-Buten, 2-Buten und Isobuten als Raffinat I, einer Fraktion aus 1-Buten, cis/trans-2-Buten, Isobuten sowie n-Butan und Isobutan, eingesetzt werden.

5. Verfahren nach Anspruch 4, wobei das Raffinat I vor der Umsetzung zur Reinigung und Adsorbermaterialien geleitet wird.

6. Verfahren nach Anspruch 4 oder 5, wobei das Verhältnis von Molanteil 1-Buten zu Molanteil 2-Buten 10:1 bis 1:10 beträgt und das Verhältnis von Molanteil 1-Buten + 2-Buten zu Isobuten 10:1 bis 1:5 beträgt.

7. Verfahren nach einem der Ansprüche 1 bis 6, wobei ein homogener oder heterogener Metathesekatalysator eingesetzt wird, der eine Rhenium- oder Rutheniumverbindung enthält.

8. Verfahren nach Anspruch 7, wobei der Metathesekatalysator Re₂O₇ auf einem Al₂O₃-Träger enthält oder daraus besteht, wobei der Rheniumoxidgehalt 1 bis 20 Gew.-%, bezogen auf das Gesamtgewicht des Katalysators, beträgt.

9. Verfahren nach einem der Ansprüche 1 bis 8, wobei die Umsetzung von 1-Buten, 2-Buten und Isobuten und/oder die Umsetzung von 2-Penten und 2-Methyl-2-buten mit Ethen als Reaktivdestillation durchgeführt werden.

## Claims

1. A process for preparing propene by
a) reaction of 1-butene, 2-butene and isobutene in a molar ratio of 1-butene + 2-butene to isobutene of from 10:1 to 1:5 to give propene, 2-pentene and 2-methyl-2-butene in the presence of a metathesis catalyst comprising at least one compound of a metal of transition group VIb, VIIb or VIII of the Periodic Table of the Elements,
b) subsequent separation of the propene and 2-pentene/2-methyl-2-butene formed,
c) subsequent reaction of the 2-pentene and 2-methyl-2-butene with ethene to give propene, 1-butene and isobutene in the presence of a metathesis catalyst comprising at least one compound of a metal of transition group VIb, VIIb or VIII of the Periodic Table of the Elements,
d) subsequent separation of the propene and 1-butene/isobutene formed,
e) subsequent return of the 1-butene and isobutene formed to step a).

2. A process as claimed in claim 1, wherein step b) is a distillation which can be carried out in a dividing wall column in which a propene-containing low-boiling phase, possibly a butene-containing intermediate-boiling phase and a 2-pentene- and 2-methyl-2-butene-containing bottom phase are obtained,
and/or
wherein step d) is a distillation which can be carried out in a dividing wall column in which a propene-containing low-boiling phase, a 1-butene- and isobutene-containing intermediate-boiling phase and possibly a 2-pentene- and 2-methyl-2-butene-containing bottom phase are obtained, wherein steps b) and d) can be carried out in a distillation column.

3. A process as claimed in claim 1 or 2, wherein the reaction in steps a) and/or c) is not carried to completion and in step b) and/or d) a C_{2/3}-containing low-boiling phase, a C₄ intermediate-boiling phase and a C₃₅-containing bottom phase are obtained,
wherein the low-boiling phases, which may be combined, are separated by distillation into C₂ and C₃ phases and the C₂ phase is returned to step c),
at least some of the intermediate-boiling phases, which may be combined, are returned to step a) and at least some of the bottom phases, which may be combined, are returned to step c).

4. A process as claimed in any of claims 1 to 3, wherein 1-butene, 2-butene and isobutene are used as raffinate I, viz. a fraction comprising 1-butene, cis/trans-2-butene, isobutene and also n-butane and isobutane.

5. A process as claimed in claim 4, wherein the raffinate I is passed over adsorber materials to purify it before the reaction.

6. A process as claimed in claim 4 or 5, wherein the molar ratio of 1-butene to 2-butene is from 10:1 to 1:10 and the molar ratio of 1-butene + 2-butene to isobutene is from 10:1 to 1:5.

7. A process as claimed in any of claims 1 to 6, wherein use is made of a homogeneous or heterogeneous metathesis catalyst comprising a rhenium or ruthenium compound.

8. A process as claimed in claim 7, wherein the metathesis catalyst comprises or consists of Re₂O₇ on an Al₂O₃ support, with the rhenium oxide content being from 1 to 20 % by weight, based on the total weight of the catalyst.

9. A process as claimed in any of claims 1 to 8, wherein the reaction of 1-butene, 2-butene and isobutene and/or the reaction of 2-pentene and 2-methyl-2-butene with ethene are carried out as a reactive distillation.

## Revendications

1. Procédé de préparation de propène par
a) réaction de 1-butène, de 2-butène et d'isobutène, le rapport molaire de la somme 1-butène + 2-butène à l'isobutène étant de 10:1 à 1:5, pour donner du propène, du 2-pentène et du 2-méthyl-2-butène, en présence d'un catalyseur de métathèse, qui contient au moins un composé d'un métal des sous-groupes Vlb., Vllb. ou du groupe VIII. de la classification périodique des éléments,
b) suivie de la séparation du propène formé et du mélange 2-pentène/2-méthyl-2-butène,
c) suivie de la réaction du 2-pentène et du 2-méthyl-2-butène avec de l'éthène pour donner du propène, du 1-butène et de l'isobutène en présence d'un catalyseur de métathèse, qui contient au moins un composé d'un métal des sous-groupes Vlb., Vllb. ou du groupe VIII. de la classification périodique des éléments,
d) suivie de la séparation du propène et du 1-butène/isobutène formés,
e) suivie du recyclage, du 1-butène et de l'isobutène formés, dans l'étape a).

2. Procédé selon la revendication 1, dans lequel l'étape b) est une distillation qui peut étre effectuée dans une colonne à cloison, permettant d'obtenir une fraction à bas point d'ébullition contenant du propène, éventuellement une fraction à point d'ébullition moyen contenant du butène et une fraction à point d'ébullition élevé contenant du 2-pentène et du 2-méthyl-2-butène,
et/ou
dans lequel l'étape d) est une distillation, qui peut être effectuée dans une colonne à cloison, permettant d'obtenir une fraction à bas point d'ébullition contenant du propène, une fraction à point d'ébullition moyen contenant du 1-butène et de l'isobutène et éventuellement une fraction à point d'ébullition élevé contenant du 2-pentène et du 2-méthyl-2-butène, les étapes b) et d) pouvant être effectuées dans une colonne de distillation.

3. Procédé selon la revendication 1 ou 2, dans lequel la réaction dans les étapes a) et/ou c) ne va pas jusqu'à son terme et dans l'étape b) et/ou d) on obtient une fraction à bas point d'ébullition en C_{2/3}, une fraction à point d'ébullition moyen en C₄ et une fraction à point d'ébullition élevé en C_{≥5},
dans lequel les fractions à bas point d'ébullition éventuellement réunies sont séparées par distillation en fractions en C₂ et fractions en C₃, et la fraction en C₂ est recyclée dans l'étape c),
les fractions à point d'ébullition moyen éventuellement réunies sont recyclées au moins en partie dans l'étape a) et les fractions à point d'ébullition élevé éventuellement réunies sont recyclées au moins en partie dans l'étape c).

4. Procédé selon l'une quelconque des revendications 1 à 3, dans lequel on met en oeuvre du 1-butène, du 2-butène et de l'isobutène en tant que raffinat l, une fraction de 1-butène, de cis/trans-2-butène, d'isobuténe et de n-butane et d'isobutane.

5. Procédé selon la revendication 4, dans lequel on fait passer le raffinat I avant la réaction sur un matériau adsorbant à des fins de purification.

6. Procédé selon la revendication 4 ou 5, dans lequel le rapport molaire du 1-butène au 2-butène est de 10:1 à 1:10 et le rapport molaire de la somme 1-butène + 2-butène à l'isobutène est de 10:1 à 1:5.

7. Procédé selon l'une quelconque des revendications 1 à 6, dans lequel on utilise un catalyseur de métathèse homogène ou hétérogène qui contient un composé du rhénium ou du ruthénium.

8. Procédé selon la revendication 7, dans lequel le catalyseur de métathèse contient, ou est constitué de, Re₂O₇ sur un support en Al₂O₃, la teneur en oxyde de rhénium étant de 1 à 20 % en poids par rapport au poids total du catalyseur.

9. Procédé selon l'une quelconque des revendications 1 à 8, dans lequel la réaction du 1-butène, du 2-butène et de l'isobutène et/ou la réaction du 2-pentène et du 2-méthyl-2-butène avec de l'éthène sont réalisées sous forme de distillation réactive.
